# FASCICULE DE BREVET EUROPEEN

(11) **EP 2 994 092 B1**
(45) Date de publication et mention de la délivrance du brevet: **02.01.2019**
(21) Numéro de dépôt: 14726426.1
(22) Date de dépôt: 06.05.2014
(51) Int. Cl.: A61K 8/43, A61K 8/58, A61K 8/67, A23L 33/12, A61K 8/27, A61K 8/31, A61Q 3/02, A61K 8/92, A61K 8/44, A61K 8/36, A61K 8/46, A61Q 3/00, A61K 8/97

(54) **ASSOCIATION D'ACTIFS POUR UNE ADMINISTRATION PAR VOIE ORALE POUR AMELIORER LA QUALITE DES ONGLES**
WIRKSTOFFKOMBINATION ZUR ORALEN VERABREICHUNG ZWECKS VERBESSERUNG DER QUALITÄT DER NÄGEL
COMBINATION OF ACTIVE AGENTS FOR ORAL ADMINISTRATION FOR IMPROVING THE QUALITY OF NAILS

(30) Priorité: 07.05.2013 FR 1354190
(43) Date de publication de la demande: 16.03.2016
(73) Titulaire: NUTRICOS Technologies, 92117 Clichy Cedex (FR)
(72) Inventeur: MAHE, Yann, F-91700 Sainte Genevieve Des Bois (FR); BRU, Carole, F-92400 Courbevoie (FR); PICCARDI, Nathalie, F-21310 Arceau (FR); GUENICHE, Audrey, F-92500 Rueil Malmaison (FR)
(74) Mandataire: Nony
(86) Numéro de dépôt international: PCT/IB2014/061247
(87) Numéro de publication internationale: WO 2014/181259

(56) Documents cités:
- EP-A1- 0 679 383
- EP-A1- 1 932 509
- WO-A2-99/40955
- WO-A2-2013/068960
- DE-A1-102005 057 292
- FR-A1- 2 939 040
- GB-A- 2 458 466
- MINTEL: "Skin, Hair and Nails Food Supplement", GNPD,, 1 novembre 2011 (2011-11-01), XP002678758,

## Description

La présente invention concerne le domaine des produits cosmétiques, pour administration par voie orale, destinés au soin des ongles.

En particulier, la présente invention vise à proposer des associations d'actifs utiles pour améliorer la qualité des ongles, diminuer et/ou éviter leurs défauts esthétiques, et/ou améliorer l'aspect esthétique général des ongles.

Plus particulièrement, la présente invention vise à proposer des associations d'actifs aptes à améliorer la microvascularisation des ongles.

Comme cela apparaitra par la suite, il est plus particulièrement question dans le cadre de la présente invention de l'amélioration de la qualité des ongles et de leur aspect esthétique général, notamment chez des individus pré-hypertendus.

Un ongle ou plaque unguéale est une lame cornée flexible, lisse et translucide, formant une excroissance superficielle de la peau, constituée par des kératinocytes et une matrice très dense et homogène de kératine. Cette dernière maintient soudées entre elles les cellules, et confère à l'ongle sa résistance, sa dureté, sa solidité et sa flexibilité. L'ongle est enveloppé d'un manchon épidermique, ou matrice.

Sur le plan morphologique, un ongle est constitué d'une partie dorsale, d'une partie intermédiaire, d'une partie ventrale, d'une matrice proximale, d'une matrice intermédiaire, d'une lunule, et du lit de l'ongle. 80 % de l'épaisseur d'un ongle est produite par la matrice proximale, et 20 % de son épaisseur est produite par la matrice intermédiaire et le lit de l'ongle. La partie dorsale est constituée de kératine dure, la partie intermédiaire est la plus épaisse et est formée de kératine moyennement dure, et la partie ventrale est constituée de kératine molle.

Le réseau microvasculaire des ongles est particulièrement dense et spécialisé. L'ongle repose sur un réseau microvasculaire en boucles, plus ou moins étirées selon la localisation anatomique de l'ongle, et constitue ainsi une zone d'échanges privilégiée pour l'approvisionnement en nutriments des ongles via le réseau sanguin.

Sur le plan de sa constitution chimique, un ongle contient de l'eau, des lipides, des mucopolysaccharides et des minéraux, tels le sodium, le potassium, le fer, le calcium, le zinc ou le silicium, et des protéines keratiniques.

La qualité générale des ongles, ainsi que leur aspect esthétique général, dépendent notamment de leur constitution chimique, en particulier de leur teneur en eau, en lipides, et en phospholipides. L'apport en nutriments au niveau des ongles est également un facteur important dans la modification de leur qualité.

Les inventeurs ont récemment démontré qu'il existe un lien statistique significatif entre la population pré-hypertendue et la qualité des ongles. En effet, il a été observé que l'état de pré-hypertension prédispose significativement à une moins bonne qualité des ongles, notamment à leur délamination et à leur casse prématurée.

Les individus pré-hypertendus présentent en effet une diminution de la fonctionnalité du réseau microvasculaire des ongles. Ainsi, les ongles de ces individus, moins vascularisés donc moins irrigués, et en conséquence moins nourris, sont affectés dans leur qualité ainsi que dans leur aspect esthétique général.

La pré-hypertension est définie comme étant la partie haute de la plage de normalité de la tension artérielle, un individu se trouvant dans cette plage étant tout de même dit « normotendu ». Ainsi, un individu pré-hypertendu n'est pas malade. On se situe en conséquence en dehors du domaine pathologique que peut représenter par exemple l'hypertension.

Un individu pré-hypertendu est défini comme ayant une pression sanguine systolique allant de 120 à 139 mmHg ou une pression sanguine diastolique allant de 80 à 89 mmHg.

A ce jour, les principales solutions proposées dans le domaine de la qualité des ongles reposent sur la mise en oeuvre de vernis à ongles, d'actifs hydratants dans les produits de soin des mains, ou d'un renforcement chimique de l'ongle. Cette dernière solution s'appuie sur l'utilisation d'agents durcisseurs pour ongles, tel que du formaldéhyde à 1-2 %, générant des liaisons croisées dans la kératine. Toutefois, un usage fréquent de ces produits peut provoquer trop de liaisons croisées, favorisant paradoxalement la fragilité des ongles.

Des implants provisoires, tels que des faux ongles, ont également été proposés dans le domaine de la qualité des ongles, mais ils ont pour principal objectif de cacher la mauvaise qualité des ongles plutôt que de l'empêcher et/ou de restaurer leur qualité. Sur un plan cosmétique, il existe donc un besoin d'être en mesure de diminuer ou d'éviter les différentes altérations esthétiques susceptibles d'affecter les ongles, notamment chez les individus pré-hypertendus, et ainsi d'améliorer la qualité des ongles et leur aspect esthétique général, et donc l'aspect esthétique des doigts, des mains et/ou des pieds.

La métalloprotéine 9 (MMP-9) est une protéase inductible et ubiquitaire capable de dégrader le collagène et l'élastine, dont le rôle dans l'altération de l'élasticité vasculaire en lien avec la pré-hypertension a été mise en évidence (Yasmin Sharon Wallace et coll. Arterioscler Thromb Vase Biol. 2005). Un lien direct entre une augmentation des taux circulants de MMP-9 et la pré-hypertension a ainsi été établi. En effet, la MMP-9 est un contributeur important à l'établissement d'une perte d'élasticité des parois vasculaires, à la distension anormale des vaisseaux, et en conséquence au développement ou au maintien d'un état de pré-hypertension.

Cette protéase intervient ainsi dans l'altération de la microvascularité des ongles chez les individus pré-hypertendus.

Il ressort donc que la MMP-9 serait une cible potentielle pour agir efficacement sur l'intégrité et la qualité du réseau microvasculaire des ongles.

La caractérisation d'actifs, voire d'une association d'actifs, aptes à inhiber l'activité de la MMP-9 permettrait ainsi de renforcer ou de restaurer le réseau microvasculaire des ongles, notamment dans le cas d'individus pré-hypertendus, permettant ainsi d'améliorer ou de maintenir la qualité des ongles et leur aspect esthétique général.

La présente invention vise précisément à répondre à ce besoin.

Les inventeurs ont ainsi découvert qu'une association comprenant au moins l'acide pétrosélinique et un composant additionnel choisi parmi la taurine, l'arginine, la cystéine, le zinc, et l'un de leurs sels, le lycopène et le zinc, en particulier les sels de Zn (II), et de préférence complexés par un ou plusieurs (poly)hydroxyacides, tel que le gluconate de zinc, s'avère, de manière surprenante, active pour inhiber la MMP-9.

L'efficacité de cette association est d'autant plus surprenante que l'acide pétrosélinique, mis en oeuvre sous une forme isolée, ne manifeste aucune activité inhibitrice à l'égard de la MMP-9, comme cela ressort de la partie expérimentale ci-après.

En revanche, l'acide pétrosélinique est déjà connu pour son efficacité pour différentes applications, telles que l'hydratation de la peau sèche dans EP 0 709 084 ou le traitement des pellicules et des démangeaisons du cuir chevelu dans EP 0 116 439.

Comme illustré dans les exemples du présent document, les inventeurs ont en effet constaté qu'une association d'actifs conforme à l'invention s'avère apte à inhiber de façon synergique le niveau basal de synthèse et/ou de libération de la MMP-9 par des kératinocytes.

Plus précisément, une association d'actifs selon l'invention permet d'améliorer efficacement la microvascularité des ongles, d'améliorer la qualité des ongles, ainsi que leur aspect esthétique général, de même que l'aspect esthétique général des doigts, des mains et/ou des pieds.

La présente invention propose en particulier une association d'acide pétrosélinique avec au moins un actif choisi parmi la taurine, l'arginine, la cystéine, et l'un de leurs sels, le lycopène et le zinc, ou l'un de ses sels, en particulier un sel de Zn (II), et de préférence complexé par un ou plusieurs (poly)hydroxyacides, tel que le gluconate de zinc, destinée à une administration par voie orale.

L'invention a donc pour objet principal l'utilisation cosmétique, par voie orale, d'une association d'actifs comprenant au moins de l'acide pétrosélinique et au moins un actif choisi parmi la taurine, l'arginine, la cystéine, le zinc, l'un de leurs sels, et le lycopène, pour améliorer la qualité des ongles, de préférence chez un individu pré-hypertendu.

La présente invention concerne également l'utilisation cosmétique, par voie orale, d'une association d'actifs selon l'invention, pour améliorer la microvascularisation des ongles.

La présente invention concerne également l'utilisation cosmétique, par voie orale, d'une association d'actifs selon l'invention, pour améliorer l'aspect esthétique général des ongles.

La présente invention concerne également l'utilisation cosmétique, par voie orale, d'une association d'actifs selon l'invention, pour améliorer l'aspect esthétique général des doigts, des mains et/ou des pieds.

L'invention vise également l'utilisation d'une association d'actifs selon l'invention, mise en oeuvre au sein d'une composition cosmétique destinée à une administration par voie orale ou au sein d'un complément alimentaire.

Une composition cosmétique destinée à une administration par voie orale ou un complément alimentaire selon l'invention confère les mêmes avantages que ceux procurés par l'association conforme à l'invention, tels qu'indiqués précédemment.

Les utilisations selon l'invention sont de préférence destinées à un individu pré-hypertendu.

Une association d'actifs, une composition ou un complément alimentaire selon l'invention peut être utilisé quotidiennement pendant plusieurs mois, sans prescription médicale. La présente invention se situe donc clairement en dehors du champ thérapeutique.

La présente invention vise en outre un kit ou ensemble de conditionnement comprenant :
(i) une association d'actifs, ou un complément alimentaire conformes à l'invention, et
(ii) un agent anti-fongique destiné à une application par voie topique,
l'association, ou le complément (i) et l'agent anti-fongique (ii) étant destinés à être administrés indépendamment l'un de l'autre, de manière séparée, simultanée ou étalée dans le temps, l'agent anti-fongique (ii) étant de préférence administré avant l'association, ou le complément (i).

La présente invention vise également un kit ou ensemble de conditionnement comprenant :
(i) une association d'actifs, ou un complément alimentaire conformes à l'invention, et
(ii) un agent hydratant et/ou un agent durcisseur destiné(s) à une application par voie topique,
l'association, ou le complément (i) et l'agent hydratant et/ou l'agent durcisseur (ii) étant destinés à être administrés indépendamment l'un de l'autre, de manière séparée, simultanée ou étalée dans le temps.

Selon un mode de réalisation, un kit selon l'invention met en oeuvre un agent anti-fongique. Un tel agent peut être choisi parmi les familles des imidazolés, des morpholines ou des pyridones.

Selon un autre mode de réalisation, un kit selon l'invention met en oeuvre un agent hydratant et/ou un agent durcisseur.

Un agent hydratant conforme à l'invention peut être choisi parmi les vitamines et les huiles. A titre d'exemple d'huile convenant à titre d'agent hydratant, peuvent notamment être cités l'huile d'argan, l'huile de graine de sésame et l'huile de tournesol.

Un agent durcisseur conforme à l'invention peut être choisi parmi les protéines hydrolysées de blé, le pantothénate de calcium ou vitamine B5, le fer, les résines époxy et polyesters, et la nitrocellulose.

Par améliorer la qualité des ongles, on entend améliorer leur dureté, et/ou leur solidité, et/ou leur résistance aux chocs et/ou aux facteurs extérieurs agressifs, et/ou leur résistance au dédoublement, et/ou leur aspect lisse, et/ou leur brillance, et/ou leur vitesse de régénération et/ou de pousse, et/ou l'homogénéité de leur couleur, et/ou leur transparence et/ou leur souplesse.

Sont particulièrement concernés par les utilisations selon l'invention les individus pré-hypertendus.

Il est entendu dans le cadre de la présente invention que « l'utilisation cosmétique par voie orale » recouvre l'utilisation de produits administrés par voie orale, ces produits étant par exemple sous forme de complément alimentaire comme exposé ci-après. Ces produits produisent un effet, au niveau des ongles, sur le plan esthétique, ou encore à visée beauté, par exemple en vue de les protéger, de les maintenir en bon état, et notamment de les embellir.

### Association d'actifs

### 1. Acide pétrosélinique

On entend par huile riche en acide pétrosélinique, une huile comprenant au moins 20 % d'acide pétrosélinique et plus préférentiellement plus de 30 % d'acide petrosélinique.

Alternativement, l'acide pétrosélinique, acide gras mono-insaturé (C18 :1 n-12 ou cis delta 6) ou acide delta-6-cis-octadecenoique en C18, est mis en oeuvre dans une association d'actifs conforme à l'invention.

Les ombellifères sont des plantes dont les fleurs sont disposées en ombelles. Les espèces particulièrement riches en acide pétrosélinique sont les Umbellifarea-Apiacea et Araliaceae. Les plantes du genre Thapsia sont également des sources d'acide pétrosélinique (Avato et al., Lipids, 2001, 36, 845). Les espèces de préférence utilisées dans l'invention sont le coriandre, le cerfeuil, la carotte, le céleri, le cumin, le carvi, le persil et l'aneth. L'huile d'ombellifère utilisée selon l'invention peut être extraite des graines de ces ombellifères, par exemple par broyage ou pressage, puis raffinage. L'huile d'ombellifère a une teneur en acide pétrosélinique qui varie selon la graine d'ombellifère dont elle est extraite. Pour une même ombellifère, la teneur en acide pétrosélinique varie aussi selon le pays d'origine de l'ombellifère et selon l'extraction qui peut être plus ou moins complète.

L'acide pétrosélinique est également un composé abondant (environ 48 %) de l'huile de graine de *Geranium sanguineum,* ainsi que de l'huile de graines de coriandre *Coriandrum sativum* (environ 65 %).

Selon un mode de réalisation, l'acide pétrosélinique est utilisé dans la présente invention sous forme isolée ou sous la forme d'un extrait végétal en contenant, notamment sous la forme d'une huile.

Ainsi, selon un mode de réalisation, l'utilisation objet de la présente invention est telle que l'acide pétrosélinique est utilisé sous forme d'huile d'ombellifère ou d'huile de *Geranium sanguineum,* de préférence sous la forme d'une huile de coriandre (*Coriandrum sativum*).

L'huile d'ombellifère est de préférence choisie parmi une huile de graines de coriandre, de cerfeuil, de carotte, de céleri, de cumin, de carvi, de persil, d'aneth, ou leurs mélanges, l'huile d'ombellifère étant de manière préférée une huile de graines de coriandre (*Coriandrum sativum*).

Les teneurs sont variables selon que l'association d'actifs conformes à l'invention est mise en oeuvre dans une composition cosmétique destinée à une administration par voie orale ou sous la forme d'un complément alimentaire.

La teneur en acide pétrosélinique, dans une composition cosmétique destinée à l'administration par voie orale ou dans un complément alimentaire conformes à l'invention, peut être comprise entre 10 et 70 % en poids, notamment entre 15 et 70 % en poids, particulièrement entre 20 et 70 % en poids, par rapport au poids total de ladite association d'actifs.

La teneur en acide pétrosélinique dans une composition cosmétique destinée à l'administration par voie orale ou dans un complément alimentaire conformes à l'invention, peut être telle que la dose journalière dudit acide pétrosélinique est comprise entre 5 et 1000 mg/j, et notamment entre 50 et 650 mg/j.

### 2. Taurine, arginine, cystéine, zinc et lycopène

Une association d'actifs selon l'invention comprend également au moins un actif choisi parmi la taurine, ou l'hypotaurine, l'arginine, la cystéine, le zinc et le lycopène.

Elle peut également mettre en oeuvre au moins l'un des sels de la taurine ou de l'hypotaurine, de l'arginine, de la cystéine ou du zinc. Dans la mesure où l'association selon l'invention est destinée à une mise en oeuvre par voie orale chez un individu, les sels pouvant être mis en oeuvre sont bien évidemment choisis pour leur totale innocuité. Conviennent à ce titre les sels alcalins ou alcalinoterreux, en particulier les sels de magnésium, manganèse, fer II ou zinc.

En ce qui concerne le zinc, conviennent notamment au sens de l'invention les sels de Zn (II) et de préférence ceux complexés par un ou plusieurs (poly)hydroxyacides.

Selon un mode de réalisation, un sel de zinc mis en oeuvre dans une association, une composition ou un complément alimentaire conformes à l'invention est le gluconate de zinc.

La teneur totale en taurine, hypotaurine, arginine et/ou cystéine, ou l'un de leurs sels, dans une composition cosmétique destinée à l'administration par voie orale ou dans un complément alimentaire conformes à l'invention, peut être comprise entre 5 et 90 % en poids, notamment entre 5 et 50 % en poids, particulièrement entre 5 et 40 % en poids, par rapport au poids total de ladite association d'actifs.

La teneur totale en taurine, hypotaurine, arginine et/ou cystéine, ou l'un de leurs sels, dans une composition cosmétique destinée à l'administration par voie orale ou dans un complément alimentaire conformes à l'invention, peut être telle que la dose journalière de ladite taurine, arginine et/ou cystéine est comprise entre 10 et 700 mg/j, et notamment entre 50 et 250 mg/j.

La teneur en zinc, ou en l'un de ses sels, en particulier en gluconate de zinc, dans une composition cosmétique destinée à l'administration par voie orale ou dans un complément alimentaire conformes à l'invention peut être comprise entre 0,001 % et 40 % en poids, notamment entre 0,01 et 40 % en poids, particulièrement entre 0,1 et 20 % en poids, par rapport au poids total de l'association d'actifs.

La teneur en zinc, ou en l'un de ses sels, notamment en gluconate de zinc, dans une composition cosmétique destinée à l'administration par voie orale ou dans un complément alimentaire conformes à l'invention, peut être telle que la dose journalière dudit zinc ou l'un de ses sels est comprise entre 0,01 et 300 mg/j, notamment entre 0,1 et 200 mg/j, particulièrement entre 1 et 100 mg/j.

Le lycopène est un pigment naturel que l'on trouve dans les fruits mûrs, particulièrement dans la tomate, ou synthétisé, notamment à partir d'un champignon, *Blakeslea trispora.* Il appartient à la famille des caroténoïdes et sa structure est proche de celle du β-carotène.

Il peut en particulier être vendu par la société Lycored sous la dénomination Lyc-O-Mato ®.

De préférence, l'association d'actifs conforme à l'invention comprend, outre de l'acide pétrosélinique, au moins du lycopène.

La teneur en lycopène dans une composition cosmétique destinée à l'administration par voie orale ou dans un complément alimentaire conformes à l'invention, peut être comprise entre 0,1 % et 30 % en poids, notamment entre 0,1 % et 20 % en poids, particulièrement entre 0,1 % et 10 % en poids, par rapport au poids total de l'association d'actifs.

La teneur en lycopène dans une composition cosmétique destinée à l'administration par voie orale, ou dans un complément alimentaire conforme à l'invention, peut être telle que la dose journalière dudit lycopène est comprise entre 0,1 et 20 mg/j, et notamment entre 0,5 et 10 mg/j.

De préférence, de la taurine, ou au moins l'un de ses sels, est mise en oeuvre dans une association d'actifs conforme à l'invention. Autrement dit, l'association d'actifs selon l'invention comprend de préférence, voire est constituée, au moins de l'acide pétrosélinique et de taurine.

De préférence, du lycopène est mis en oeuvre dans une association d'actifs conforme à l'invention. Autrement dit, l'association d'actifs selon l'invention comprend de préférence au moins de l'acide pétrosélinique et du lycopène.

De préférence, de l'arginine est mise en oeuvre dans une association d'actifs conforme à l'invention. Autrement dit, l'association d'actifs selon l'invention comprend de préférence au moins de l'acide pétrosélinique et de l'arginine.

De préférence, du zinc ou l'un de ses sels est mis en oeuvre dans une association d'actifs conforme à l'invention. Autrement dit, l'association d'actifs selon l'invention comprend de préférence au moins de l'acide pétrosélinique et du zinc ou l'un de ses sels, de préférence du gluconate de zinc.

De préférence, de la cystéine est mise en oeuvre dans une association d'actifs conforme à l'invention. Autrement dit, l'association d'actifs selon l'invention comprend de préférence au moins d'acide pétrosélinique et de la cystéine.

Une composition, ou un complément alimentaire selon l'invention, peut également comprendre au moins un agent choisi parmi un tripeptide d'hydrolysat de lait Val-Pro-Pro et/ou Ile-Pro-Pro, un concentré aqueux de tomate, un flavonoïde, du CoQ10, de l'acétyle carnitine, de l'acide alpha lipoïque et de la citrulline.

Selon un mode de réalisation, une composition ou un complément alimentaire selon l'invention comprend du zinc ou l'un de ses sels.

Comme indiqué précédemment, le sel de zinc préféré selon l'invention est le gluconate de zinc.

Ainsi, selon un mode de réalisation préféré de l'invention, une composition, un complément alimentaire selon l'invention comprend de l'acide pétrosélinique, de la taurine et du zinc, en particulier du gluconate de zinc.

Selon un mode de réalisation, une composition cosmétique destinée à une administration par voie orale ou complément alimentaire conforme à l'invention comprend :
(i) de l'acide pétrosélinique, en une teneur comprise entre 10 et 70 % en poids, notamment entre 15 et 70 % en poids, particulièrement entre 20 et 70 % en poids, par rapport au poids total de l'association d'actifs ;
(ii) de la taurine, en une teneur comprise entre 5 et 90 % en poids, notamment entre 5 et 50 % en poids, particulièrement entre 5 et 40 % en poids, par rapport au poids total de l'association d'actifs ; et
(iii) optionnellement du zinc ou l'un de ses sels, de préférence un (poly)hydroxyacide de zinc, préférentiellement du gluconate de zinc, en une teneur comprise entre 0,001 et 40 % en poids, notamment entre 0,01 et 40 % en poids, particulièrement entre 0,1 et 20 % en poids, par rapport au poids total de l'association d'actifs ;
(iv) optionnellement de la vitamine D3, en une teneur comprise entre 0,0001 et 1,0 % en poids, notamment entre 0,0001 et 0,5 % en poids, particulièrement entre 0,0001 et 0,1 % en poids, par rapport au poids total de l'association d'actifs ; et/ou
(v) optionnellement de la vitamine E ou l'un de ses dérivés, de préférence de l'acétate de tocophérol, en une teneur comprise entre 0,01 et 10 % en poids, notamment entre 0,1 et 10 % en poids, particulièrement entre 0,2 et 5 % en poids, par rapport au poids total de l'association d'actifs.

Selon un mode de réalisation particulier, une composition cosmétique destinée à une administration par voie orale ou un complément alimentaire conformes à l'invention comprend les ingrédients i) et ii) et optionnellement l'ingrédient iii) indiqués précédemment.

Selon un mode de réalisation particulier, une composition cosmétique destinée à une administration par voie orale ou un complément alimentaire conformes à l'invention comprend les ingrédients i) à v) suivants, pris ensembles ou indépendamment :
(i) de l'acide pétrosélinique, en une teneur comprise entre 1 et 70 % en poids, notamment entre 10 et 70 % en poids, particulièrement entre 20 et 70 % en poids par rapport au poids total de la composition ou du complément ;
(ii) de la taurine, en une teneur comprise entre 5 et 90 % en poids, notamment entre 5 et 50 % en poids, particulièrement entre 5 et 40 % en poids, par rapport au poids total de la composition ou du complément ; et/ou
(iii) au moins un (poly)hydroxyacide de zinc, de préférence du gluconate de zinc, en une teneur comprise entre 0,001 et 40 % en poids, notamment entre 0,01 et 40 % en poids, particulièrement entre 0,1 et 20 % en poids, par rapport au poids total de la composition ou du complément ;
(iv) optionnellement de la vitamine D3, en une teneur comprise entre 0,0001 et 1,0 % en poids, notamment entre 0,0001 et 0,5 % en poids, particulièrement entre 0,0001 et 0,1 % en poids, par rapport au poids total de la composition ou du complément ; et/ou
(v) optionnellement de la vitamine E ou l'un de ses dérivés, de préférence de l'acétate de tocophérol, en une teneur comprise entre 0,01 et 10 % en poids, notamment entre 0,1 et 10 % en poids, particulièrement entre 0,2 et 5 % en poids, par rapport au poids total de la composition ou du complément.

Selon un mode de réalisation particulier, la composition cosmétique destinée à une administration par voie orale ou le complément alimentaire conformes à l'invention comprend l'ensemble des ingrédients (i) à (v) susmentionnés.

Une telle composition de type complément alimentaire ou composition orale conforme à l'invention peut en particulier présenter les teneurs suivantes :

| **Composants** | **% en poids par rapport au poids total de la composition** |
|---|---|
| Acide pétrosélinique | 54,9 *(apporté par l'huile de graines de coriandre)* |
| Gluconate de zinc | 6,3 (dont 13,6 % de matière active) |
| Taurine | 18,7 (dont 98,5 % de matière active) |
| Vitamine E | 1,0 (dont 67 % de matière active) |
| Vitamine D3 | 0,03 (dont 2,5 % de matière active) |

Pour l'ingestion, de nombreuses formes de réalisation de compositions orales, et notamment de compléments alimentaires sont possibles. Leur formulation est réalisée par les procédés usuels pour produire des dragées, gélules, gels, émulsions, comprimés, tablettes ou capsules molles.

Selon la présente invention, une composition cosmétique destinée à l'administration par voie orale, ou un complément alimentaire conformes à l'invention peut en outre comprendre au moins une vitamine choisie parmi la vitamine B1, B3, B5, B6, B8, B9, B12, C, D, et notamment D3, et le tocophérol (vitamine E) et ses dérivés, notamment ester tel que l'acétate ou le palmitate de tocophérol, de préférence de l'acétate de tocophérol.

Selon un mode de réalisation, une composition cosmétique destinée à une administration par voie orale ou un complément alimentaire conformes à la présente invention tel(le) que décrit(e) plus haut comprend de préférence au moins de la vitamine E ou l'un de ses dérivés et/ou de la vitamine D, préférentiellement de la vitamine D3 et/ou de l'acétate de tocophérol.

Selon un mode de réalisation particulier, une composition cosmétique destinée à une administration par voie orale ou un complément alimentaire conformes à la présente invention tel(le) que décrit(e) plus haut comprend de la vitamine D3 et de l'acétate de tocophérol.

Ainsi, selon un mode de réalisation, une composition cosmétique destinée à l'administration par voie orale, ou un complément alimentaire conformes à l'invention comprend de l'acide pétrosélinique, de la taurine, du zinc ou l'un de ses sels, de préférence du gluconate de zinc, de la vitamine D3 et de l'acétate de tocophérol.

Les compositions ou compléments alimentaires selon l'invention peuvent en outre comprendre au moins un actif choisi parmi de la glucosamine, du collagène ou de l'acide hyaluronique.

Les compositions ou compléments alimentaires selon l'invention peuvent en outre comprendre au moins un actif choisi parmi de l'extrait de romarin, de l'acide rosmarinique, de l'acide carnosique, la curcumine, de l'extrait d'écorce de pin, du pycnogenol, de la berberine, de l'extrait de Boswellia, de l'émodine, du sésamol, du sulforaphane, de l'extrait de brocoli, du resvératrol, de l'extrait de raisin, du 6-Shogaol, de l'extrait de cassis, de l'extrait d'aubergine, de l'entérolactone, de l'extrait de nèfle du japon, de l'oleuropéine, de l'acide pachymique, du ptérostilbène, de l'hydroxytyrosol et les acides gras polyinsaturés et mono insaturées PUFA et MUFA, oméga 3 et oméga 6.

Les compositions orales ou les compléments alimentaires selon l'invention peuvent en outre comprendre au moins un probiotique, un prébiotique ou un mélange de probiotiques et un mélange de prébiotiques. A titre de microorganismes probiotiques, on peut notamment citer *Lactobacillus johnsonii* ou *Lactobacillus paracaseï.*

Les compositions selon l'invention, destinées à une administration par voie orale, peuvent comprendre l'intégralité ou une partie seulement de la dose journalière.

Autrement dit, une à trois compositions peuvent être administrées par jour.

Typiquement, la durée de ce traitement cosmétique destinée à une administration par voie orale peut être supérieure à 4 semaines, notamment de 4 à 24 semaines, avec le cas échéant une ou plusieurs périodes d'interruption.

D'autres caractéristiques et avantages de l'invention ressortiront mieux des exemples qui suivent, donnés à titre illustratif et non limitatif.

### Exemple 1

### Caractérisation des ongles fragilisés/dédoublés versus ongles normaux en relation avec un état de pré-hypertension.

Soixante-dix femmes en bonne santé, âgées de 18 à 50 ont été incluses dans l'étude indiquée dans la suite du présent texte. Sur ces 70 femmes, 35 avaient des ongles fragilisés/dédoublés et 35 des ongles normaux.

Le caractère fragilisé des ongles a été confirmé par une évaluation clinique utilisant l'échelle publiée précédemment par Van de Kerkhof (**van de Kerkhof PC, Pasch MC, Scher RK, Kerscher M, Gieler U, Haneke E, Fleckman P.** Brittle nail syndrome: a pathogenesis-based approach with a proposed grading system. J Am Acad Dermatol. 2005 Oct;53(4):644-51*.),* échelle qui prend en compte l'onychoschizie (dédoublement transversal et lamellaire) et l'onychorrexie (stries et cassures longitudinales) des ongles testés. Ces évaluations ont été réalisées sur l'ongle le plus abimé choisi par le clinicien sur l'une des deux mains.

Au cours de cette étude, une mesure de la pression artérielle a été effectuée selon une méthode bien connue de l'homme de métier.

De manière toute à fait surprenante, il a été mis en évidence une différence statistiquement significative de la pression systolique chez les sujets présentant des ongles fragilisés/dédoublés en comparaison de la population présentant des ongles normaux.

| | Ongles normaux (n=42) | Ongles fragilisés/dédoublés (n=42) | Comparaison inter-groupe (p-value) t-test |
|---|---|---|---|
| Pression systolique (mmHg) | 114.929 ± 10.538 | 121.238 ± 10.529 | **S, p=0.007** |

L'état de pré-hypertension correspond dans JNC VII à une pression systolique comprise entre 120-139 mmHg (JNC VII Express. The seventh report of the joint national committee on prevention, detection, evaluation and treatment of high blood pressure. National Institute of Health; Washington DC 2003; Publication n°03-5233). La population de femmes présentant des ongles fragilisés/dédoublés incluses dans cette étude peut donc être considérée comme une population saine présentant une pré-hypertension.

### Exemple 2

### Mise en évidence de l'effet inhibiteur d'associations d'actifs selon l'invention sur la synthèse/libération basale de MMP-9 par des kératinocytes épidermiques humains.

De nombreux types cellulaires sont capables de produire de la MMP-9 : les cellules endothéliales, les cellules immunitaires, les cellules conjonctives et les kératinocytes.

Les kératinocytes cutanés humains ont été choisis comme modèle expérimental d'évaluation *in vitro* en raison de leur grande disponibilité et de leur facilité de mise en oeuvre, ainsi que pour leur analogie avec les cellules endothéliales dans la mesure où elles contribuent à la formation d'épithéliums.

En effet, les cellules endothéliales capillaires sont plus difficiles à obtenir. De même, leur grande sensibilité aux protéases ne permet pas, contrairement aux kératinocytes en culture, de tester simplement et de façon reproductible l'effet des agents modulateurs de ces MMPs. Cela est notamment dû à des effets inhibiteurs sur la croissance de ces cellules des produits de dégradation (angiostatines) produits par la plupart des MMPs (Brauer et al. BMC Biochemistry 2011, 12:38).

Par ailleurs, dans l'ongle, comme dans le cheveu, il existe une forte proximité anatomique entre les kératinocytes et les vaisseaux, suggérant que les kératinocytes peuvent aussi être une des sources de production de MMP-9 capable d'altérer la physiologie des vaisseaux et des ongles.

Des actifs conformes à l'invention sous forme isolée ou non ont été testés sur des kératinocytes épidermiques humains normaux (NHEK) afin de déterminer leur effet sur le niveau basal de synthèse et/ou de libération de la métalloprotéinase 9 (MMP-9) par ces cellules.

Le Tableau I ci-après rend compte de la nature des composés testés (seuls ou en association - 1^{ère} colonne) et des résultats obtenus (2^{ème} colonne).

Ce test a été réalisé sur des cellules cultivées à 37°C, 5 % CO₂. Le milieu de culture est du Keratinocyte-SFM complémenté avec du facteur de croissance épidermique (EGF) à 0,25 ng/ml, de l'extrait puituitaire (EP) à 25 µg/ml et de la gentamycine à 25 µg/ml.

Les kératinocytes ont été cultivés dans le milieu de culture indiqué ci-dessus pendant 24 h. Les cellules ont ensuite été traitées, ou non (pour le témoin), par les composés ou associations de composés indiquées dans le Tableau I ci-après, et incubées pendant 24 h aux doses indiquées dans le Tableau I.

A la fin de cette incubation, les surnageants de culture ont été collectés et conservés afin de doser les quantités de MMP-9 sécrétées à l'aide du kit de dosage ELISA R&D Systems de Référence DY911.

Les comparaisons intergroupes ont été réalisées à l'aide du test T de Student bilatéral non apparié.

Les variations de quantités mesurées sont indiquées dans le Tableau I ci-après.

**Tableau I**

| **Composés/Associations de composés testés** | **% de synthèse/libération basale de MMP-9 par des kératinocytes** |
|---|---|
| Acide pétrosélinique seul (0,1 µM) | -12 % (ns) |
| Arginine seule (1,7 µg/ml) | -11 % (ns) |
| **Arginine (1,7 µg/ml) + Acide pétrosélinique (0,1 µM)** | **-33 %*** |
| Taurine seule (1,2 µg/ml) | +11 % (ns) |
| **Taurine (1,2 µg/ml) + Acide pétrosélinique (0,1 µM)** | **-27 %*** |
| Cystéine seule (1,2 µg/ml) | -12 % (ns) |
| **Cystéine (1,2 µg/ml) + Acide pétrosélinique (0,1 µM)** | **-30 %*** |
| Lyc-o-mato seul (10⁻⁵ %) | +26 % (ns) |
| **Lycomato (10⁻⁵ %) + Acide pétrosélinique (0,1 µM)** | **-24 %*** |

| | |
|---|---|
| (ns) : non significatif par rapport au niveau basal de synthèse et/ou de libération de la MMP-9. * : significatif par rapport au niveau basal de synthèse et/ou de libération de la MMP-9. | |

Tout d'abord, on peut constater que l'ensemble des composés testés, lorsqu'ils sont mis en oeuvre individuellement, n'inhibent pas de façon significative le niveau basal de synthèse et/ou de libération de la MMP-9. Certains composés (le lycomato et la taurine) présentent même une tendance inverse et modérée, sans que celle-ci ne soit cependant significative.

Au contraire, les associations d'actifs conformes à l'invention présentent bien une inhibition significative du niveau basal de synthèse et/ou de libération de la MMP-9.

En effet, on peut observer une diminution de la quantité de MMP-9 dans le milieu de culture des kératinocytes traités par rapport à la quantité présente en l'absence de traitement, ou même en présence de chacun des éléments testés pris individuellement. Cela indique en conséquence une diminution du niveau basal de synthèse et/ou de libération de la MMP-9 par ces cellules en présence d'une association d'actifs selon l'invention.

C'est donc bien un effet synergique des associations conformes à l'invention qui est ici observé et démontré. En effet, des actifs n'ayant aucun effet individuellement sur la synthèse et/ou la libération de la MMP-9 par des kératinocytes présentent toutefois un tel effet lorsqu'ils sont associés conformément à la présente invention.

### Exemple 3

**Composition pour la voie orale sous forme de capsule molle.**

| **Ingrédients** | **(mg/capsule molle)** |
|---|---|
| Huile de graines de coriandre (65 % acide pétrosélinique) | 300 |
| Taurine | 76,10 |
| Gluconate de zinc | 25,75 |
| Vitamine E | 4,10 |
| Vitamine D3 | 0,115 |

| **Excipients** | |
|---|---|
| Huile de coco, raffinée | 112 |
| Cire d'abeille, Jaune, Cera flava | 22 |
| Lécithine de tournesol | 10 |

| **Capsule** | |
|---|---|
| Gélatine de poisson | 144,6 |
| Glycérol | 58,6 |
| Eau purifiée | 6,8 |

Lorsque cette composition est administrée à un individu par voie orale, à raison de deux capsules par jour, on peut observer une amélioration de la qualité et de l'aspect esthétique général des ongles chez cet individu.

Ces effets sont encore plus visibles lorsque l'individu testé est pré-hypertendu, dans la mesure où ses ongles sont, avant le test, d'une faible qualité, et présentent un faible, voire un mauvais aspect esthétique général.

Une association d'actifs selon l'invention permet un meilleur apport en nutriments par le sang des cellules perfusées par ces micro-vaisseaux ainsi protégées vis-à-vis des atteintes liées à l'activité des MMP-9, et en conséquence une meilleure qualité ainsi qu'un meilleur aspect esthétique général de l'ongle et de la peau qui l'entoure.

### Exemple 4

**Composition pour la voie orale sous forme d'émulsion en stick.**

| **Ingrédients** | **(g/stick)** |
|---|---|
| Huile de graines de coriandre (dont 65 % d'acide pétroselinique) | 0,65 |
| Taurine | 0,0515 |
| Vitamine E | 0,0082 |
| Lycopène | 0,005 |

| **Excipients** | |
|---|---|
| Eau | 1,722 |
| Sucre | 0,911 |
| Fructose | 0,911 |
| Microcristalline cellulose | 0,032 |
| Carboxymethyl cellulose sodique | 0,004 |
| Mélange naturel de tocophérols | 0,034 |
| Huile de tournesol | 3,015 |
| Arôme naturel de citron | 0,034 |
| Sorbate de potassium | 0,013 |
| Acide citrique | 0,013 |
| Alginate de propylène glycol | 0,010 |

### Exemple 5

**Composition pour la voie orale sous forme de capsule molle.**

| **Ingrédients** | **(mg/capsule molle)** |
|---|---|
| Huile de graines de coriandre (65% acide petroselinique) | 300 |
| Arginine | 82 |
| Cystéine | 25 |
| Vitamine E | 4,10 |

| **Excipients** | |
|---|---|
| Huile de coco, raffinée | 112 |
| Cire d'abeille, jaune, Cera flava | 22 |
| Lécithine de tournesol | 10 |

| **Capsule** | |
|---|---|
| Gélatine de poisson | 144,6 |
| Glycérol | 58,6 |
| Eau purifiée | 6,8 |

## Revendications

1. Utilisation cosmétique, par voie orale, d'une association d'actifs comprenant au moins de l'acide pétrosélinique et au moins un actif choisi parmi la taurine, l'arginine, la cystéine, le zinc, l'un de leurs sels, et le lycopène, afin d'améliorer la dureté, et/ou la solidité, et/ou la résistance aux chocs et/ou aux facteurs extérieurs agressifs, et/ou la résistance au dédoublement, et/ou l'aspect lisse, et/ou la brillance et/ou la vitesse de régénération et/ou de pousse, et/ou l'homogénéité de la couleur, et/ou la transparence, et/ou la souplesse des ongles.

2. Utilisation cosmétique, par voie orale, selon la revendication 1 d'une association d'actifs comprenant au moins de l'acide pétrosélinique et au moins un actif choisi parmi la taurine, l'arginine, la cystéine, le zinc, l'un de leurs sels, et le lycopène, pour améliorer la qualité de la microvascularisation des ongles.

3. Utilisation selon l'une quelconque des revendications précédentes, pour améliorer l'aspect esthétique général des ongles.

4. Utilisation selon l'une quelconque des revendications précédentes, destinée à un individu pré-hypertendu.

5. Utilisation selon l'une quelconque des revendications précédentes, dans laquelle ledit acide pétrosélinique est utilisé sous une forme isolée ou sous la forme d'un extrait végétal en contenant, notamment sous la forme d'une huile.

6. Utilisation selon l'une quelconque des revendications précédentes, dans laquelle ledit acide pétrosélinique est utilisé sous forme d'huile d'ombellifère ou de *Geranium sanguineum.*

7. Utilisation selon la revendication 6, dans laquelle ladite huile d'ombellifère est choisie parmi les huiles de graines de coriandre, de cerfeuil, de carotte, de céleri, de cumin, de carvi, de persil, d'aneth, et leurs mélanges, l'huile d'ombellifère étant de préférence sous la forme d'une huile de graines de coriandre.

8. Utilisation selon l'une quelconque des revendications précédentes, dans laquelle l'association d'actifs comprend au moins de l'acide pétrosélinique et de la taurine.

9. Utilisation selon l'une quelconque des revendications précédentes, dans laquelle l'association d'actifs comprend au moins de l'acide pétrosélinique et du lycopène.

10. Utilisation selon l'une quelconque des revendications précédentes, dans laquelle l'association d'actifs comprend au moins de l'acide pétrosélinique et de l'arginine.

11. Utilisation selon l'une quelconque des revendications précédentes, dans laquelle l'association d'actifs comprend au moins de l'acide pétrosélinique et du zinc, en particulier un sel de Zn (II), et de préférence complexé par un ou plusieurs (poly)hydroxyacides, tel que le gluconate de zinc.

12. Utilisation selon l'une quelconque des revendications précédentes, dans laquelle l'association d'actifs comprend au moins de l'acide pétrosélinique et de la cystéine.

13. Utilisation selon l'une quelconque des revendications précédentes, dans laquelle l'association d'actifs est mise en oeuvre au sein d'un complément alimentaire.

14. Utilisation selon la revendication 13, dans laquelle le complément alimentaire comprend en outre au moins un agent choisi parmi un tripeptide d'hydrolysat de lait Val-Pro-Pro et/ou Ile-Pro-Pro, un concentré aqueux de tomate, un flavonoïde, du CoQ10, de l'acétyle camitine, de l'acide alpha lipoïque et de la citrulline.

15. Utilisation selon la revendication 13 ou 14, dans laquelle ledit complément alimentaire comprend :
(i) de l'acide pétrosélinique, sous forme isolée ou sous la forme d'une huile d'ombellifère, et plus particulièrement sous la forme d'une huile de graines de coriandre, en une teneur comprise entre 10 et 70 % en poids, notamment entre 15 et 70 % en poids, particulièrement entre 20 et 70 % en poids du poids total de l'association d'actifs ;
(ii) de la taurine, ou l'un de ses sels, en une teneur comprise entre 5 et 90 % en poids, notamment entre 5 et 50 % en poids, particulièrement entre 5 et 40 % en poids du poids total de l'association d'actif ; et
(iii) optionnellement du zinc, ou l'un de ses sels, de préférence du gluconate de zinc, en une teneur comprise entre 0,001 et 40 % en poids, notamment entre 0,01 et 40 % en poids, particulièrement entre 0,1 et 20 % en poids, du poids total de l'association d'actifs.

16. Utilisation selon l'une quelconque des revendications 14 à 15, dans laquelle le complément alimentaire comprend en outre au moins une vitamine choisie parmi la vitamine B1, B3, B5, B6, B8, B9, B12, C, D, de préférence D3, ou la vitamine E et ses dérivés, et comprend de préférence de la vitamine D et/ou de la vitamine E ou l'un de ses dérivés, préférentiellement de la vitamine D3 et/ou de la vitamine E ou l'un de ses dérivés, de préférence de la vitamine D3 et/ou de l'acétate de tocophérol, plus préférentiellement de la vitamine D3 et de l'acétate de tocophérol.

17. Utilisation selon la revendication 16, dans laquelle le complément alimentaire comprend de l'acide pétrosélinique, de la taurine, du gluconate de zinc, de la vitamine D3 et de l'acétate de tocophérol.

18. Kit comprenant :
(i) une association d'actifs, telle que définie dans l'une quelconque des revendications 1 à 12, ou un complément alimentaire tel que défini dans l'une quelconque des revendications 13 à 17 et 19, et
(ii) un agent anti-fongique destiné à une application par voie topique,
l'association, ou le complément (i) et l'agent anti-fongique (ii) étant destinés à être administrés indépendamment l'un de l'autre, de manière séparée, simultanée ou étalée dans le temps, l'agent anti-fongique (ii) étant de préférence administré avant l'association, ou le complément (i).

19. Kit comprenant :
(i) une association d'actifs, telle que définie dans l'une quelconque des revendications 1 à 12, ou un complément alimentaire tel que défini dans l'une quelconque des revendications 13 à 17 et 19, et
(ii) un agent hydratant et/ou un agent durcisseur destiné(s) à une application par voie topique,
l'association ou le complément (i) et l'agent hydratant et/ou l'agent durcisseur (ii) étant destinés à être administrés indépendamment l'un de l'autre, de manière séparée, simultanée ou étalée dans le temps.

## Patentansprüche

1. Orale kosmetische Verwendung einer Kombination von Wirkstoffen, umfassend mindestens Petroselinsäure und mindestens einen Wirkstoff, ausgewählt aus Taurin, Arginin, Cystein, Zink, einem ihrer Salze und Lycopin, zur Verbesserung der Härte und/oder Festigkeit, und/oder Beständigkeit gegen Stöße und/oder aggressive äußere Faktoren und/oder Beständigkeit gegen Spaltung und/oder des glatten Aussehens und/oder des Glanzes und/oder der Regenerations- und/oder Wachstumsgeschwindigkeit und/oder der Farbeinheitlichkeit und/oder Transparenz und/oder Geschmeidigkeit der Nägel.

2. Orale kosmetische Verwendung einer Kombination von Wirkstoffen nach Anspruch 1, die mindestens Petroselinsäure und mindestens einen Wirkstoff ausgewählt aus Taurin, Arginin, Cystein, Zink, einem Salz davon und Lycopin umfasst, um die Qualität der Mikrovaskularisation der Nägel zu verbessern.

3. Verwendung nach einem der vorangehenden Ansprüche, um das allgemeine ästhetische Aussehen der Nägel zu verbessern.

4. Verwendung gemäß einem der vorangehenden Ansprüche, die für eine prähypertensive Person bestimmt ist.

5. Verwendung nach einem der vorangehenden Ansprüche, wobei die Petroselinsäure in isolierter Form oder in Form eines sie enthaltenden Pflanzenextrakts, insbesondere in Form eines Öls, verwendet wird.

6. Verwendung nach einem der vorangehenden Ansprüche, wobei die Petroselinsäure in Form von Doldenblütleröl oder *Geranium sanguineum* verwendet wird.

7. Verwendung nach Anspruch 6, wobei das Doldenblütleröl ausgewählt ist aus Koriandersamenöl, Kerbelöl, Karottenöl, Sellerieöl, Kreuzkümmelöl, Kümmelöl, Petersilienöl, Dillöl und Mischungen davon, wobei das Doldenblütleröl vorzugsweise in Form eines Koriandersamenöls vorliegt.

8. Verwendung nach einem der vorangehenden Ansprüche, wobei die Kombination von Wirkstoffen mindestens Petroselinsäure und Taurin umfasst.

9. Verwendung nach einem der vorangehenden Ansprüche, wobei die Kombination von Wirkstoffen mindestens Petroselinsäure und Lycopin umfasst.

10. Verwendung nach einem der vorangehenden Ansprüche, wobei die Kombination von Wirkstoffen mindestens Petroselinsäure und Arginin umfasst.

11. Verwendung nach einem der vorangehenden Ansprüche, wobei die Kombination von Wirkstoffen mindestens Petroselinsäure und Zink, insbesondere ein Salz von Zn(II), und komplexiert vorzugsweise mit einer oder mehreren (Poly)hdroxysäuren, wie beispielsweise Zinkgluconat, umfasst.

12. Verwendung nach einem der vorangehenden Ansprüche, wobei die Kombination von Wirkstoffen mindestens Petroselinsäure und Cystein umfasst.

13. Verwendung nach einem der vorangehenden Ansprüche, wobei die Kombination von Wirkstoffen in einem Nahrungsergänzungsmittel durchgeführt wird.

14. Verwendung nach Anspruch 13, wobei das Nahrungsergänzungsmittel ferner mindestens ein Mittel umfasst, ausgewählt aus einem Tripeptid von Milchhydrolysat Val-Pro-Pro und/oder Ile-Pro-Pro, einem wässrigen Tomatenkonzentrat, einem Flavonoid, CoQlO, Acetylcarnitin, Alpha-Liponsäure und Citrullin.

15. Verwendung nach Anspruch 13 oder 14, wobei das Nahrungsergänzungsmittel umfasst:
i) Petroselinsäure in isolierter Form oder in Form von Doldenblütleröl, insbesondere in Form von Koriandersamenöl, in einem Gehalt von 10 bis 70 Gew.-%, insbesondere von 15 bis 70 Gew.-%, insbesondere von 20 bis 70 Gew.-% des Gesamtgewichts der Kombination von Wirkstoffen;
ii) Taurin oder ein Salz davon in einem Gehalt von 5 bis 90 Gew.-%, insbesondere von 5 bis 50 Gew.-%, insbesondere von 5 bis 40 Gew.-% des Gesamtgewichts der Kombination von Wirkstoffen; und
(iii) gegebenenfalls Zink oder ein Salz davon, vorzugsweise Zinkgluconat, in einem Gehalt von 0,001 bis 40 Gew.-%, insbesondere von 0,01 bis 40 Gew.-%, insbesondere von 0,1 bis 20 Gew.-%, des Gesamtgewichts der Kombination von Wirkstoffen.

16. Verwendung nach einem der Ansprüche 14 bis 15, wobei das Nahrungsergänzungsmittel ferner mindestens ein Vitamin umfasst, ausgewählt aus Vitamin B1, B3, B5, B5, B6, B8, B9, B12, C, D, vorzugsweise D3, oder Vitamin E und seinen Derivaten, und vorzugsweise Vitamin D und/oder Vitamin E oder eines seiner Derivate, vorzugsweise Vitamin D3 und/oder Vitamin E oder eines seiner Derivate, vorzugsweise Vitamin D3 und/oder Tocopherolacetat, vorzugsweise Vitamin D3 und Tocopherolacetat umfasst.

17. Verwendung nach Anspruch 16, wobei das Nahrungsergänzungsmittel Petroselinsäure, Taurin, Zinkgluconat, Vitamin D3 und Tocopherolacetat umfasst.

18. Kit umfassend:
(i) eine Kombination von Wirkstoffen nacheinem der Ansprüche 1 bis 12 oder ein Nahrungsergänzungsmittel nach einem der Ansprüche 13 bis 17 und 19, und
(ii) ein Antimykotikum zur topischen Anwendung,
wobei die Kombination oder das Ergänzungsmittel (i) und das Antimykotikum (ii) unabhängig voneinander zur getrennten, gleichzeitigen oder zeitverzögerten Verabreichung bestimmt sind, wobei das Antimykotikum (ii) vorzugsweise vor der Kombination oder dem Ergänzungsmittel (i) verabreicht wird.

19. Kit umfassend:
(i) eine Kombination von Wirkstoffen nach einem der Ansprüche 1 bis 12 oder ein Nahrungsergänzungsmittel nach einem der Ansprüche 13 bis 17 und 19 und
(ii) ein Feuchthaltemittel und/oder ein Härtungsmittel zur topischen Anwendung,
wobei die Kombination oder das Ergänzungsmittel (i) und das Feuchthaltemittel und/oder Härtungsmittel (ii) unabhängig voneinander zur getrennten, gleichzeitigen oder zeitverzögerten Verabreichung bestimmt sind.

## Claims

1. Cosmetic use, via oral route, of an association of active agents comprising at least petroselinic acid and at least one active agent selected from among taurine, arginine, cysteine, zinc, one of their salts, and lycopene to improve nail hardness and/or solidity, and/or resistance to shocks and/or to harsh external factors, and/or resistance to splitting, and/or the smooth appearance and/or sheen and/or rate of regeneration and/or growth, and/or colour uniformity, and/or transparency and/or flexibility of nails.

2. The cosmetic use via oral route according to claim 1 of an association of active agents comprising at least petroselinic acid and at least one active agent selected from among taurine, arginine, cysteine, zinc, one of their salts and lycopene, to improve the quality of nail micro-vascularisation.

3. The use according to any of the preceding claims, to improve the general cosmetic appearance of nails.

4. The use according to any of the preceding claims, intended for a pre-hypertensive individual.

5. The use according to any of the preceding claims, wherein said petroselinic acid is used in isolated form or in the form of a plant extract containing the same, in particular in oil form.

6. The use according to any of the preceding claims, wherein said petroselinic acid is used in the form of an umbellifer plant oil or *Geranium sanguineum* oil.

7. The use according to claim 6, wherein said umbellifer oil is selected from among coriander seed, chervil, carrot, celery, cumin, caraway, parsley, dill oils and mixtures thereof, the umbellifer oil preferably being in the form of coriander seed oil.

8. The use according to any of the preceding claims, wherein the association of active agents comprises at least petroselinic acid and taurine.

9. The use according to any of the preceding claims, wherein the association of active agents comprises at least petroselinic acid and lycopene.

10. The use according to any of the preceding claims, wherein the association of active agents comprises at least petroselinic acid and arginine.

11. The use according to any of the receding claims, wherein the association of active agents comprises at least petroselinic acid and zinc, in particular a Zn (II) salt, and preferably complexed with one or more polyhydroxy acids such as zinc gluconate.

12. The use according to any of the preceding claims, wherein the association of active agents comprises at least petroselinic acid and cysteine.

13. The use according to any of the preceding claims, wherein the association of active agents is used in a food supplement.

14. The use according to claim 13, wherein the food supplement also comprises at least one agent selected from among a Val-Pro-Pro and/or Ile-Pro-Pro milk hydrolysate tripeptide, an aqueous tomato concentrate, a flavonoid, CoQ10, acetyl carnitine, alpha lipoic acid and citrulline.

15. The use according to claim 13 or 14, wherein said food supplement comprises:
(i) petroselinic acid in isolated form or in the form of an umbellifer oil, and more particularly in the form of coriander seed oil, in a content of between 10 and 70 weight %, in particular between 15 and 70 weight %, particularly between 20 and 70 weight % of the total weight of the association of active agents;
(ii) taurine, or a salt thereof, in a content of between 5 and 90 weight %, in particular between 5 and 50 weight %, particularly between 5 and 40 weight % of the total weight of the association of active agents; and
(iii) optionally zinc, or a salt thereof, preferably zinc gluconate, in a content of between 0.001 and 40 weight %, in particular between 0.01 and 40 weight %, particularly between 0.1 and 20 weight % of the total weight of the association of active agents.

16. The use according to any of claims 14 to 15, wherein the food supplement also comprises at least one vitamin selected from among vitamin B1, B3, B5, B6, B8, B9, B12, C, D, preferably D3, or vitamin E and the derivatives thereof, and preferably comprises vitamin D and/or vitamin E or one of the derivatives thereof, preferably vitamin D3 and/or vitamin E or one of the derivatives thereof, preferably vitamin D3 and/or tocopherol acetate, more preferably vitamin D3 and tocopherol acetate.

17. The use according to claim 16, wherein the food supplement comprises petroselinic acid, taurine, zinc gluconate, vitamin D3 and tocopherol acetate.

18. Kit comprising:
(i) an association of active agents such as defined in any of claims 1 to 12, or a food supplement such as defined in any of claims 13 to 17 and 19; and
(ii) an anti-fungal agent intended to be applied via topical route,
the association or supplement (i) and anti-fungal agent (ii) being intended to be administered independently of each other, separately, simultaneously or extended over time, the anti-fungal agent (ii) preferably being administered before the association or the supplement (i).

19. Kit comprising:
(i) an association of active agents such as defined in any of claims 1 to 12, or a food supplement such as defined in any of claims 13 to 17 and 19; and
(ii) a hydrating agent and/or hardening agent intended to be applied via topical route,
the association or supplement (i) and hydrating agent and/or hardening agent (ii) being intended to be administered independently of each other, separately, simultaneously or extended over time.
